# FASCICULE DE BREVET EUROPEEN

(11) **EP 2 780 319 B1**
(45) Date de publication et mention de la délivrance du brevet: **30.03.2016**
(21) Numéro de dépôt: 12787015.2
(22) Date de dépôt: 16.11.2012
(51) Int. Cl.: C07C 255/32, C07C 255/37, C07D 211/88, C07C 253/00

(54) **PROCEDE DE FABRICATION DE COMPOSES COMPRENANT DES FONCTIONS NITRILES**
VERFAHREN ZUR HERSTELLUNG VON VERBINDUNGEN MIT NITRILFUNKTIONEN
METHOD FOR PRODUCING COMPOUNDS COMPRISING NITRILE FUNCTIONS

(30) Priorité: 18.11.2011 FR 1160534
(43) Date de publication de la demande: 24.09.2014
(73) Titulaire: RHODIA OPERATIONS, 93306 Aubervilliers (FR)
(72) Inventeur: MARION, Philippe, F-69390 Vernaison (FR); JACQUOT, Roland, F-69340 Francheville (FR); GRIMAUD, Laurence, F-75003 Paris (FR); CARTIGNY, Damien, F-17000 La Rochelle (FR); ELKAIM, Laurent, F-75012 Paris (FR)
(74) Mandataire: Chatelan, Florence Anne
(86) Numéro de dépôt international: PCT/EP2012/072849
(87) Numéro de publication internationale: WO 2013/072466

(56) Documents cités:
- WO-A1-2011/144619
- FR-A1- 2 922 887
- DAVID A. KLEIN: "Nitrile synthesis via the acid-nitrile exchange reaction", THE JOURNAL OF ORGANIC CHEMISTRY, vol. 36, no. 20, 1 octobre 1971 (1971-10-01), pages 3050-3051, XP055014514, ISSN: 0022-3263, DOI: 10.1021/jo00819a035
- LAECKMANN D ET AL: "Synthesis and biological evaluation of aroylguanidines related to amiloride as inhibitors of the human platelet Na<+>/H<+> exchanger", BIOORGANIC & MEDICINAL CHEMISTRY, PERGAMON, GB, vol. 10, no. 6, 1 janvier 2002 (2002-01-01), pages 1793-1804, XP002429666, ISSN: 0968-0896, DOI: 10.1016/S0968-0896(02)00022-6

## Description

La présente invention concerne la fabrication de composés comprenant des fonctions nitriles et de composés imide cycliques.

Elle se rapporte plus particulièrement à la fabrication de composés comprenant des fonctions nitriles à partir de composés comprenant des fonctions carboxyliques, avantageusement d'origine naturelle et renouvelable, et à partir d'un mélange N de dinitriles comprenant du méthyl-2 glutaronitrile (MGN), de l'éthyl-2 succinonitrile (ESN) et de l'adiponitrile (AdN).

Les composés comprenant des fonctions nitriles sont des produits importants pour la fabrication de composés amines. Les composés dinitriles conduisent à des amines qui sont par exemple des monomères à l'origine de polymères tels que le polyamide, par exemple. Les composés mononitriles conduisent à des amines ou à des amides qui sont par exemple utilisés pour la fabrication de tensioactifs cationiques.

De nombreux procédés de synthèse de nitriles ont été proposés, notamment des procédés de synthèse à partir d'ammoniac et d'acides carboxyliques. Ces procédés utilisent principalement comme matière première de départ des composés hydrocarbures issus du raffinage du pétrole, et de l'ammoniac, qui est obtenu à partir d'hydrogène provenant de procédés de vaporéformage qui consomment du gaz et de l'énergie.

Klein, J. Org. Chem. 1971, page 3050, décrit un procédé de synthèse de nitriles catalysé par des acides.

Compte tenu de l'épuisement de la ressource pétrolière, de nombreux travaux de recherche sont entrepris pour développer des procédés de synthèse de ces composés, importants pour la fabrication de matériaux utilisés dans de nombreuses applications, à partir de matières premières ou ressources dites renouvelables, ou à partir de matières premières recyclées, qui sont habituellement détruites ou valorisées uniquement sous forme d'énergie. Généralement ces ressources renouvelables sont produites à partir de matière végétale cultivée ou non telle que les arbres, les plantes comme la canne à sucre, le maïs, le manioc, le blé, le colza, le tournesol, la palme, le ricin ou analogues, ou à partir de matière animale telle que les graisses (suif etc.).

Cette matière végétale ou animale est transformée par des procédés comprenant généralement plusieurs étapes mécaniques, chimiques voire biologiques.

Par ailleurs, à propos des matières premières recyclées, la fabrication de l'adiponitrile, un grand intermédiaire chimique utilisé notamment dans la synthèse de l'hexaméthylène diamine et du caprolactame (monomères pour la fabrication des polyamides), obtenu par hydrocyanation du butadiène, génère un flux de sous-produits dinitriles comprenant majoritairement des composés dinitriles ramifiés comme le méthyl-2 glutaronitrile, l'éthyl-2 succinonitrile. Ce mélange de composés dinitriles ramifiés est obtenu par distillation pour le séparer de l'adiponitrile. Comme la séparation n'est généralement pas complète, le mélange de composés dinitriles ramifiés peut comprendre également une faible proportion d'adiponitrile.

Plusieurs solutions ont été proposées pour valoriser ces sous-produits ou mélanges. Une de celles-ci consiste à hydrogéner les composés dinitriles en amines primaires notamment pour produire de la méthyl-2 pentaméthylènediamine (MPMD), utilisée comme monomère pour la fabrication de polyamides particuliers ou comme intermédiaire pour la production de vitamine B3 (nicotinamide).
Ce procédé requiert des étapes de purification du méthyl-2 glutaronitrile et de la méthyl-2 pentaméthylènediamine.

Dans l'industrie, ces sous-produits sont également valorisés sous forme de vapeur ou d'énergie par combustion. Toutefois, cette combustion peut demander un traitement des gaz pour éliminer les oxydes d'azote produits et elle produit du gaz carbonique qui est rejeté dans l'atmosphère.

Il existe donc un besoin et une demande importante de trouver de nouvelles voies de valorisation et transformation de ces composés dinitriles ou des mélanges en composés chimiques valorisables et économiquement intéressants.

A cet effet, l'invention propose un procédé de préparation d'au moins un nitrile de formule générale I

(NC)ᵥ-R₁-(CN)_{w}

ou respectivement d'au moins un nitrile de formule générale III 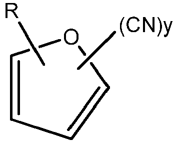 ou respectivement d'au moins un nitrile de formule générale V 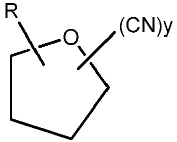 et d'au moins l'imide cyclique 3-méthyl-glutarimide,
par réaction entre au moins un acide carboxylique de formule générale II

(HOOC)ₓ-R₁-(COOH)_{z}

ou respectivement au moins un acide carboxylique de formule générale IV 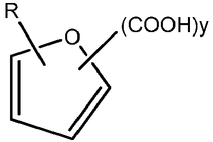 ou respectivement au moins un acide carboxylique de formule générale VI 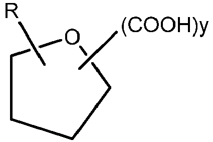 et au moins le méthyl-2 glutaronitrile (MGN), en présence d'un catalyseur acide de Lewis
avec
x, z est égal à 0 ou 1 avec (x+z) égal à 1 ou 2
v, w est égal à 0 ou 1 avec (v+w) égal à 1 ou 2
R₁ représente un groupement hydrocarboné linéaire ou ramifié, saturé ou insaturé, pouvant comprendre des hétéroatomes, comprenant :
   - de 4 à 34 atomes de carbone lorsque (x+z) est égal à 2,
   - de 2 à 22 atomes de carbone lorsque (x+z) est égal à 1
y est égal à 1 ou 2
R symbolise un ou plusieurs substituants

L'acide carboxylique de formule IV ou VI peut être porteur d'un ou plusieurs substituants.

Avantageusement on met en oeuvre un mélange N de dinitriles comprenant du méthyl-2 glutaronitrile (MGN), de l'éthyl-2 succinonitrile (ESN) et de l'adiponitrile (AdN). On obtient alors au moins les imides cycliques 3-méthyl-glutarimide et 3-éthylsuccinimide.

De préférence le mélange N de dinitriles est un mélange issu du procédé de fabrication de l'adiponitrile par double hydrocyanation du butadiène. Il correspond de préférence à la fraction de distillation permettant de séparer les dinitriles ramifiés (méthyl-2 glutaronitrile, éthyl-2 succinonitrile) de l'adiponitrile.

Ce mélange de dinitriles a généralement la composition pondérale suivante :
Méthyl-2 glutaronitrile : comprise entre 70 % et 95 %, de préférence entre 80 et 85%
Ethyl-2 succinonitrile : compris entre 5 % et 30 %, de préférence entre 8 et 12%
Adiponitrile : compris entre 0 % et 10 %, de préférence entre 1 et 5%
le complément à 100% correspondant à différentes impuretés

Le procédé de l'invention met en oeuvre un acide carboxylique de formule générale II, IV ou VI telles que décrites ci-dessus.

Avantageusement l'acide carboxylique de formule générale II, IV ou VI est issu d'une matière renouvelable d'origine végétale ou animale.

Une matière ou ressource renouvelable est une ressource naturelle, animale ou végétale, dont le stock peut se reconstituer sur une période courte à l'échelle humaine. Il faut en particulier que ce stock puisse se renouveler aussi vite qu'il est consommé.

A la différence des matériaux issus de matières fossiles, les matières premières renouvelables contiennent une proportion importante de ¹⁴C. De préférence les nitriles de l'invention sont constitués de carbone organique issu de matières premières renouvelables. Ainsi cette caractéristique préférée pourrait être certifiée par détermination de la teneur en ¹⁴C selon l'une des méthodes décrites dans la norme ASTM D6866, notamment selon la méthode par spectrométrie de masse ou la méthode par spectrométrie à scintillation liquide qui sont décrites dans cette norme.

Ces ressources renouvelables peuvent être produites à partir de matière végétale cultivée ou non telle que les arbres, les plantes comme la canne à sucre, le maïs, le manioc, le blé, le colza, le tournesol la palme, le ricin ou analogues, ou à partir de matière animale telle que les graisses (suif etc.).

Par exemple, l'acide carboxylique de formule générale II, IV ou VI peut être issu de ressources renouvelables tels que les huiles végétales ou les polysaccharides naturels comme par exemple l'amidon ou la cellulose, l'amidon pouvant être extrait, par exemple, du maïs, du blé ou de la pomme de terre. Il peut en particulier provenir de divers procédés de transformation, notamment de procédés chimiques classiques, de procédés de transformation par voie enzymatique ou encore par fermentation.

Lorsque le composé de formule Il est un monoacide gras, ce dernier peut par exemple être obtenu à partir d'huile végétale ou animale par transformation chimique (hydrolyse des huiles).

Lorsque le composé de formule Il est un diacide, ce dernier peut être obtenu par fermentation à partir d'un monoacide gras obtenu selon la méthode ci-dessus. Par exemple, il est possible d'utiliser la levure *Candida Tropicalis* modifiée afin de réaliser la conversion d'un monoacide en diacide. On pourra notamment se référer aux documents WO 91/06660 et US 4474882. Le diacide peut également être obtenu à partir d'huile végétale ou animale par transformation chimique.

Lorsque la matière première est un polysaccharide, le composé de formule Il est généralement obtenu par fermentation.

L'acide 2,5-furannedicarboxylique peut par exemple être obtenu à partir de l'acide mucique ou à partir de l'hydroxyméthylfurfural.

Le radical R₁ peut être un radical aliphatique, un groupement comprenant un radical aromatique ou cycloaliphatique, il peut être fonctionnalisé par exemple par une fonction hydroxyle, une fonction ester etc.

Le composé de formule Il peut par exemple être choisi parmi l'acide trichloroacétique, l'acide trifluoroacétique, l'acide caproïque, l'acide caprylique, l'acide pélargonique, l'acide caprique, l'acide laurique, l'acide myristique, l'acide palmitique, l'acide stéarique, l'acide arachidique, l'acide adipique, l'acide heptanedioïque, l'acide octanedioïque, l'acide azélaïque, l'acide sébacique, ou undécanedioïque, l'acide dodécanedioïque, l'acide brassylique, l'acide tetradécanedioïque, l'acide hexadécanedioïque, l'acide octadécanedioïque, l'acide eicosanedioïque, l'acide docosanedioïque, l'acide octadécènoïque, l'acide oléique, l'acide ricinoléique, l'acide érucique, l'acide linoléique, l'acide linolénique et les dimères d'acides gras contenant 36 atomes de carbone, l'acide téréphtalique, l'acide isophtalique.

Avantageusement le composé de formule Il est choisi parmi l'acide caproïque, l'acide caprylique, l'acide pélargonique, l'acide caprique, l'acide laurique, l'acide myristique, l'acide palmitique, l'acide stéarique, l'acide arachidique, l'acide adipique, l'acide heptanedioïque, l'acide octanedioïque, l'acide azélaïque, l'acide sébacique, ou undécanedioïque, l'acide dodécanedioïque, l'acide brassylique, l'acide tetradécanedioïque, l'acide hexadécanedioïque, l'acide octadécanedioïque, l'acide eicosanedioïque, l'acide docosanedioïque, l'acide octadécènoïque, l'acide oléique, l'acide ricinoléique, l'acide érucique, l'acide linoléique, l'acide linolénique et les dimères d'acides gras contenant 36 atomes de carbone, l'acide téréphtalique, l'acide isophtalique.

L'acide azélaïque peut être obtenu à partir de l'acide oléïque, par ozonolyse.

L'acide heptanedioïque et l'acide sébacique peuvent être obtenus à partir d'huile de ricin.

L'acide dodécanedioïque peut être obtenu par bio-fermentation de l'acide dodécanoïque, également dénommé acide laurique, l'acide laurique pouvant être produit à partir d'huile de noix de coco ou d'huile de palme-kernel.

L'acide brassylique peut être obtenu à partir de l'acide érucique (notamment par ozonolyse), étant précisé que l'acide érucique se trouve sous forme d'ester dans le colza.

L'acide tetradécanedioïque peut être obtenu par bio-fermentation de l'acide myristique, l'acide myristique pouvant être produit à partir d'huile de noix de coco ou d'huile de palme-kernel.

L'acide hexadécanedioïque peut être obtenu par bio-fermentation de l'acide palmitique, ce dernier se trouvant dans l'huile de palme principalement.

L'acide octadécanedioïque peut être obtenu par bio-fermentation de l'acide stéarique, étant précisé que l'acide stéarique peut être présent dans toutes les huiles végétales mais surtout dans les graisses animales.

L'acide eicosanedioïque peut être obtenu par bio-fermentation de l'acide arachidique que l'on trouve majoritairement dans l'huile de colza.

L'acide docosanedioïque peut être obtenu par métathèse de l'acide undécylénique que l'on extrait de l'huile de ricin.

Le diacide linéaire aliphatique ayant 36 atomes de carbones est un dimère d'acide gras issu par exemple des sous-produits des résineux transformés par les procédés Kraft. Il peut également être obtenu par oligomérisation ou polymérisation d'acides gras monobasiques insaturés à longue chaîne hydrocarbonée (tels que l'acide linoléïque et l'acide oléïque), comme décrit notamment dans le document EP 0471566.

Avantageusement R est choisi parmi :
- les groupes alkyle linéaires ou ramifiés ayant de préférence de 1 à 6 atomes de carbone et encore plus préférentiellement de 1 à 4 atomes de carbone,
- les groupes alkyle mono-, poly- ou perhalogénés, linéaires ou ramifiés ayant de préférence de 1 à 6 atomes de carbone et de 1 à 13 atomes d'halogène et encore plus préférentiellement de 1 à 4 atomes de carbone et de 1 à 9 atomes d'halogène,
- les groupes éther R₂-O- ou thioéther R₂-S- dans lesquels R₂ représente un groupe alkyle linéaire ou ramifié ayant de 1 à 6 atomes de carbone et encore plus préférentiellement de 1 à 4 atomes de carbone ou le groupe phényle ,
- les groupes acyloxy ou aroyloxy R₂-CO-O- dans lesquels le groupe R₂ a la signification donnée précédemment,
- les groupes acyle ou aroyle R₂-CO- dans lesquels le groupe R₂ a la signification donnée précédemment,
- le groupe hydroxyle,
- un atome d'halogène, de préférence, un atome de fluor.

Selon un mode de réalisation particulier de l'invention, y est égal à 1 et R représente -CO-O-R₃ avec R₃ qui représente un groupe alkyle ayant de 1 à 4 atomes de carbone dans la formule générale IV ou VI.

De préférence le composé de formule IV ou VI est choisi parmi l'acide 2,5-furannedicarboxylique, l'acide 2,5-tetrahydrofurannedicarboxylique, l'acide 2-furoïque, l'acide 2,5-furannedicarboxylique monométhylester ou les acides 2,5-tetrahydrofurannedicarboxylique monométhylester.

Dans le cadre du procédé de l'invention, on peut mettre en oeuvre un mélange de plusieurs acides carboxyliques, par exemple un acide carboxylique de formule générale Il ou IV ou VI et un autre acide carboxylique, ou un mélange de plusieurs acides carboxyliques de formule Il (ou IV ou VI). A titre d'exemple de mélange d'acides, on peut citer les cocoacides qui sont issus de l'huile de palme ou de l'huile de coco, les acides de suif etc.

La réaction de l'invention est réalisée en présence d'un catalyseur acide de Lewis. A titre d'exemple d'acide de Lewis, on peut citer AlCl₃, FeCl₃, ZnCl₂, TiCl₄, les triflates de lanthanides tel que Yb(OT_{f})₃.

La quantité de catalyseur mise en oeuvre est avantageusement comprise entre 0,1 et 4% molaire, de préférence entre 0,5 et 2% molaire, par rapport au nombre de moles de réactifs mis en oeuvre.

Le milieu réactionnel peut contenir des traces d'eau.

Le procédé de l'invention est avantageusement mis en oeuvre à une température comprise entre 150 et 350°C. La pression mise en oeuvre est généralement comprise entre la pression atmosphérique et quelques bars.

Le procédé de l'invention peut également être réalisé par application de micro-ondes sur le mélange réactionnel, comme alternative au chauffage traditionnel. La durée d'irradiation est avantageusement comprise entre 10 et 30 minutes, de préférence entre 5 et 15 minutes.

De façon avantageuse, on met en oeuvre une quantité de méthyl-2 glutaronitrile (MGN) ou de mélange N de telle sorte qu'au moins une molécule de méthyl-2 glutaronitrile (MGN) ou d'éthyl-2 succinonitrile (ESN) est introduite dans le milieu réactionnel, par fonction acide de l'acide carboxylique de formule générale Il ou IV ou VI à transformer en fonction nitrile.

Lorsque l'on met en oeuvre un diacide comme acide de formule générale Il ou IV ou VI, on peut obtenir le dinitrile correspondant ou le nitrile acide correspondant (par exemple en mettant en oeuvre un défaut de fonction nitrile).

Lors de la réaction entre le composé de formule (II) ou (IV) ou (VI) et le mélange N de dinitriles conformément à l'invention, des imides sont formés, notamment le 3-méthyl glutarimide issu du MGN et le 3-éthylsuccinimide issu de l'ESN.

Avantageusement, le procédé de l'invention comprend également une étape de récupération d'au moins le nitrile de formule (I) ou (III) ou (V) d'une part, et d'au moins l'imide cyclique d'autre part, à partir du milieu réactionnel.

Cette récupération peut être réalisée par séparation des composés du milieu réactionnel, selon toute méthode connue telle que la distillation.

Selon un premier mode de réalisation avantageux, les composés peuvent être obtenus par distillation réactive. En effet lorsque le nitrile de formule (I) ou (III) ou (V) que l'on souhaite obtenir a une température d'ébullition inférieure à celle de la température de réaction (ce qui est en particulier le cas pour les nitriles ayant un faible nombre de carbones), on peut distiller ce nitrile au fur et à mesure de sa formation, ce qui déplace l'équilibre de la réaction en faveur de la formation de ce nitrile ; ceci est donc particulièrement avantageux. Cette méthode de distillation réactive peut par exemple être utilisée lorsque le nitrile de formule (I) ou (III) ou (V) est le 2,5-dicyanotetrahydrofuranne, ou un mononitrile tel que l'octanitrile ou le nonanitrile.

Selon un deuxième mode de réalisation avantageux, les composés peuvent être séparés par extraction à l'eau chaude. En effet les imides sont généralement solubles dans l'eau, contrairement notamment aux nitriles, ce qui permet une bonne séparation par une voie aisée à mettre en oeuvre. Cette voie est à privilégier notamment lorsque les nitriles et les imides à séparer ont des températures d'ébullition qui sont proches et qu'ils sont par ce fait difficile à séparer par distillation classique par exemple. Cette méthode d'extraction à l'eau chaude peut par exemple être utilisée lorsque le nitrile de formule (I) est le lauronitrile, l'oléonitrile ou lorsque l'on met en oeuvre un mélange d'acides de formule (II). La température de l'eau lors de cette extraction est généralement supérieure ou égale à 50°C.

Selon un mode de réalisation particulier de l'invention, le nitrile de formule (I) ou (III) ou (V) ainsi récupéré est hydrogéné pour former l'amine correspondante, selon une méthode connue de l'homme du métier. Lorsque l'on récupère un nitrile de formule (III), son hydrogénation peut conduire à l'amine correspondant au composé de formule (V), par hydrogénation non seulement de la fonction nitrile, mais également par hydrogénation des doubles liaisons du cycle furannique. Ainsi on obtient une amine dont tous les carbones sont biosourcés (car issus d'un acide carboxylique biosourcé, c'est-à-dire un acide carboxylique issu d'une matière première renouvelable), et dont les atomes d'azote sont recyclés (car issus de sous-produits habituellement brûlés ce qui génère du dioxyde de carbone et des oxydes d'azote, gaz à effet de serre qu'il faut traiter pour satisfaire la législation en vigueur). Les diamines peuvent être utilisées comme matières premières pour la fabrication des polyamides, qui seront ainsi partiellement ou complètement biosourcés en fonction des acides utilisés pour la polymérisation. Les amines peuvent également être utilisées pour préparer des tensioactifs.

Selon un autre mode de réalisation particulier de l'invention, on peut faire réagir l'imide cyclique récupéré selon le procédé de l'invention, avec un alcool pour former le diester correspondant. Un tel procédé est connu et notamment décrit dans le document WO2008/009792 et WO 2009/056477. Les diesters peuvent être utilisés comme solvants.

D'autres détails ou avantages de l'invention apparaîtront plus clairement à la vue des exemples donnés ci-dessous.

### EXEMPLES

Dans les exemples, la réaction suivante est mise en oeuvre : 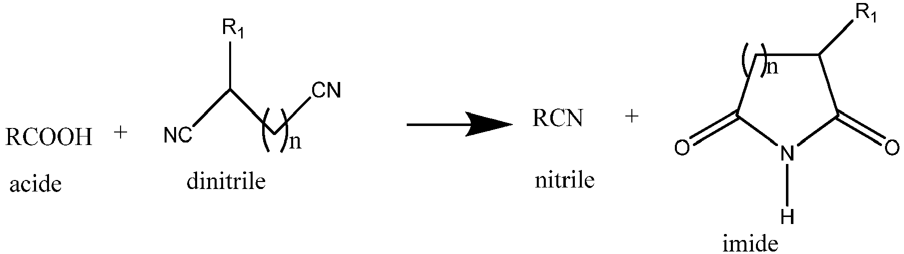 avec n=2
R1 = H ou CH3

Le tableau ci-dessous indique les conditions opératoires des exemples ainsi que les résultats.

**Tableau**

| **Exemples** | **Acide** | **Dinitrile** | **Catalyseur % molaire** | **Mode** | **TT% acide** | **RR% nitrile** | **RR% imide** |
|---|---|---|---|---|---|---|---|
| 1 comparatif | Composé 1 | glutaronitrile | - | thermique | 44 | 40 | 42 |
| 1 | Composé 1 | glutaronitrile | AlCl 3 | thermique | 85 | 83 | 84 |
| | | | 2% | | | | |
| 2 | Composé 1 | glutaronitrile | Yb(OTf)3 | thermique | 90 | 69 | 85 |
| | | | 1% | | | | |
| 3 | Composé 1 | glutaronitrile | ZnCl2 | thermique | 68 | 66 | 67 |
| | | | 1% | | | | |
| 2 comparatif | Composé 1 | glutaronitrile | - | micro-ondes | 74 | 62 | 68 |
| 4 | Composé 1 | glutaronitrile | AlCl3 | micro-ondes | 85 | 75 | 80 |
| | | | 1% | | | | |
| 5 | Composé 1 | glutaronitrile | Znl2 | micro-ondes | 86 | 86 | 86 |
| | | | 1% | | | | |
| 6 | Composé 1 | méthyl-2 glutaronitrile | AlCl3 | thermique | 84 | 76 | 83 |
| | | | 1% | | | | |
| 7 | 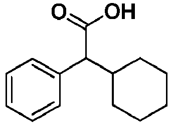 | glutaronitrile | AlCl3 | micro-ondes | 83 | 83 | 83 |
| | | | 1% | | | | |
| 8 | 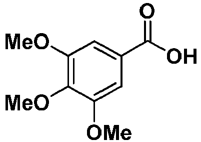 | glutaronitrile | AlCl3 | micro-ondes | 57 | 52 | 55 |
| | | | 1% | | | | |
| 9 | 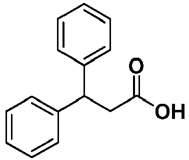 | glutaronitrile | AlCl3 | micro-ondes | 84 | 80 | 82 |
| | | | 1% | | | | |
| 10 | 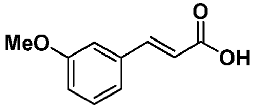 | glutaronitrile | AlCl3 | micro-ondes | 66 | 40 | 65 |
| | | | 1% | | | | |

Le composé 1 dans le tableau est le suivant :

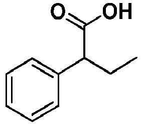

Le pourcentage molaire de catalyseur dans le tableau est exprimé par rapport à la somme molaire des réactifs acide et dinitrile

Les deux modes opératoires thermique et micro-ondes indiqués dans le tableau sont décrits ci-dessous.

### Mode thermique :

Dans un tube en verre, le dinitrile (3,0 mmol, 1,0 équiv.) est ajouté à un mélange d'acide (3,0 mmol) et de catalyseur (0,06 mmol, 0,02 équiv, sauf pour l'exemple 1 où l'on met en oeuvre 0,12 mmol, 0,04 équiv). Le tube est alors hermétiquement fermé (à l'aide d'un bouchon à vis) et laissé sous agitation magnétique à 200 °C pendant 5 h. Après réaction, le brut réactionnel est transféré dans un ballon de 50 mL avec 10 mL d'éthanol. Si nécessaire, le tube est placé dans un bain à ultra-sons à 60 °C, afin de favoriser la solubilisation du mélange réactionnel dans l'éthanol. À ce mélange sont ensuite ajoutés 3 g de silice, afin de réaliser un dépôt solide après évaporation de l'éthanol. Enfin, le nitrile pur est obtenu après chromatographie sur colonne de silice (gradient de M₁ à M₉ puis M₀). L'imide cyclique est quant à lui obtenu après élution avec de l'acétate d'éthyle. La conversion en nitrile désiré est déterminée par RMN ¹H du brut. Les rendements indiqués sont les rendements des produits isolés après purification.

### Mode micro-ondes :

Dans un tube en verre prévu à cet effet, le dinitrile (3,0 mmol, 1.0 équiv.) est ajouté à un mélange d'acide (3,0 mmol) et de catalyseur (0,06 mmol, 0,02 équiv.). Le tube est alors fermé à l'aide d'un bouchon adapté, puis il est laissé sous activation micro-ondes (appareil utilisé : **Monowave® 300 d'Anton Paar** et agitation magnétique à 300 °C pendant 10 mn. Après réaction, le brut réactionnel est transféré dans un ballon de 50 mL avec 10 mL d'éthanol. Si nécessaire, le tube est placé dans un bain à ultra-sons à 60 °C, afin de favoriser la solubilisation du mélange réactionnel dans l'éthanol. À ce mélange sont ensuite ajoutés 3 g de silice, afin de réaliser un dépôt solide après évaporation de l'éthanol. Enfin, le nitrile pur est obtenu après chromatographie sur colonne de silice (gradient de M₁ à M₉ puis M₀). L'imide cyclique est quant à lui obtenu après élution avec de l'acétate d'éthyle. La conversion en nitrile désiré est déterminée par RMN ¹H du brut. Les rendements indiqués sont les rendements des produits isolés après purification.

### Note :

M₀ est un mélange dichlorométhane/acétate d'éthyle 80:20.
M₁ est un mélange éther de pétrole/M₀ 90:10,
M₂ est un mélange éther de pétrole/M₀ 80:20, etc.
M₉ est un mélange éther de pétrole/M₀ 10:90.

### Exemple 6 comparatif

Dans un tricol de 100ml, on introduit 200 mmol de 2-méthylglutaronitrile et on ajoute 200 mmol d'acide 2-phénylbutanoique. On agite et ajoute à la suspension 4 mmol d'acide orthophosphorique à 85%. On chauffe alors le milieu réactionnel à 200°C pendant 5 heures. Après ce temps, le milieu réactionnel est analysé et on obtient les résultats suivants
TT% du MGN = 52% , RR% en MGI = 51%, RR% en 2-phénylbutanenitrile = 45%

### Exemple 11

Dans un tricol de 100ml, on introduit 200 mmol de 2-méthylglutaronitrile et 100mmol d'acide dodecanedioique. On ajoute à la suspension 3mmol d'AlCl3 anhydre. On chauffe alors à 200°C en agitant. On maintient dans ces conditions pendant 5 heures. On analyse ensuite le milieu réactionnel et on obtient les résultats suivants
TT%du MGN = 90%, RR% du MGI = 88% , RR% en dodecanedinitrile = 81%

### Exemple 12

Dans un réacteur en verre de 250ml, on introduit 130g (1200 mmol) de 2-méthylglutaronitrile, 20g (120 mmol) d'acide isophtalique. On agite la suspension blanche et on ajoute 0,32g (2,4 mmol) de chlorure d'aluminium anhydre. On chauffe progressivement à 270°C et on maintient dans ces conditions pendant 4h.
Pendant le montée en température, l'acide isophtalique se dissout dans le MGN.
On analyse ensuite par CPG le milieu réactionnel. On obtient un RR% en MGI de 76% et un rendement en 1,3-dicyanobenzène de 69%.

## Revendications

1. Procédé de préparation d'au moins un nitrile de formule générale I
(NC)ᵥ-R₁-(CN)_{w}
ou respectivement d'au moins un nitrile de formule générale III 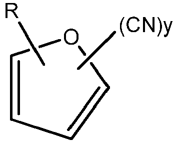 ou respectivement d'au moins un nitrile de formule générale V 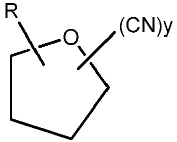 et d'au moins l'imide cyclique 3-méthyl-glutarimide,
par réaction entre au moins un acide carboxylique de formule générale II
(HOOC)ₓ-R₁-(COOH)_{z}
ou respectivement au moins un acide carboxylique de formule générale IV 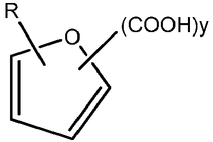 ou respectivement au moins un acide carboxylique de formule générale VI 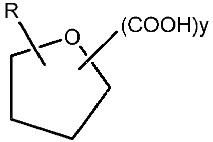 et au moins le méthyl-2 glutaronitrile (MGN), en présence d'un catalyseur acide de Lewis avec
x, z est égal à 0 ou 1 avec (x+z) égal à 1 ou 2
v, w est égal à 0 ou 1 avec (v+w) égal à 1 ou 2
R₁ représente un groupement hydrocarboné linéaire ou ramifié, saturé ou insaturé, pouvant comprendre des hétéroatomes, comprenant :
- de 4 à 34 atomes de carbone lorsque (x+z) est égal à 2,
- de 2 à 22 atomes de carbone lorsque (x+z) est égal à 1
y est égal à 1 ou 2
R symbolise un ou plusieurs substituants

2. Procédé selon la revendication 1, **caractérisé en ce que** l'on met en oeuvre un mélange N de dinitriles comprenant du méthyl-2 glutaronitrile (MGN), de l'éthyl-2 succinonitrile (ESN) et de l'adiponitrile (AdN)

3. Procédé selon la revendication 1, **caractérisé en ce que** le mélange N de dinitriles est un mélange issu du procédé de fabrication de l'adiponitrile par double hydrocyanation du butadiène.

4. Procédé selon la revendication 2 ou 3, **caractérisé en ce que** le mélange N de dinitriles a la composition pondérale suivante :
Méthyl-2 glutaronitrile : comprise entre 70 % et 95 %, de préférence entre 80 et 85%
Ethyl-2 succinonitrile : compris entre 5 % et 30 %, de préférence entre 8 et 12%
Adiponitrile : compris entre 0 % et 10 %, de préférence entre 1 et 5%
le complément à 100% correspondant à différentes impuretés

5. Procédé selon l'une des revendications précédentes, **caractérisé en ce que** le composé de formule II, IV et/ou VI est issu d'une matière renouvelable d'origine végétale ou animale

6. Procédé selon l'une des revendications précédentes, **caractérisé en ce que** le composé de formule Il est choisi parmi l'acide caproïque, l'acide caprylique, l'acide pélargonique, l'acide caprique, l'acide laurique, l'acide myristique, l'acide palmitique, l'acide stéarique, l'acide arachidique, l'acide adipique, l'acide heptanedioïque, l'acide octanedioïque, l'acide azélaïque, l'acide sébacique, ou undécanedioïque, l'acide dodécanedioïque, l'acide brassylique, l'acide tetradécanedioïque, l'acide hexadécanedioïque, l'acide octadécanedioïque, l'acide eicosanedioïque, l'acide docosanedioïque, l'acide octadécènoïque, l'acide oléique, l'acide ricinoléique, l'acide érucique, l'acide linoléique, l'acide linolénique et les dimères d'acides gras contenant 36 atomes de carbone, l'acide téréphtalique.

7. Procédé selon l'une des revendications précédentes, **caractérisé en ce que** R est choisi parmi :
- les groupes alkyle linéaires ou ramifiés ayant de préférence de 1 à 6 atomes de carbone et encore plus préférentiellement de 1 à 4 atomes de carbone,
- les groupes alkyle mono-, poly- ou perhalogénés, linéaires ou ramifiés ayant de préférence de 1 à 6 atomes de carbone et de 1 à 13 atomes d'halogène et encore plus préférentiellement de 1 à 4 atomes de carbone et de 1 à 9 atomes d'halogène,
- les groupes éther R₂-O- ou thioéther R₂-S- dans lesquels R₂ représente un groupe alkyle linéaire ou ramifié ayant de 1 à 6 atomes de carbone et encore plus préférentiellement de 1 à 4 atomes de carbone ou le groupe phényle ,
- les groupes acyloxy ou aroyloxy R₂-CO-O- dans lesquels le groupe R₂ a la signification donnée précédemment,
- les groupes acyle ou aroyle R₂-CO- dans lesquels le groupe R₂ a la signification donnée précédemment,
- le groupe hydroxyle,
- un atome d'halogène, de préférence, un atome de fluor.

8. Procédé selon l'une des revendications 1 à 6, **caractérisé en ce que** y est égal à 1 et R représente -CO-O-R₃ avec R₃ qui représente un groupe alkyle ayant de 1 à 4 atomes de carbone dans la formule générale IV ou VI

9. Procédé selon l'une des revendications précédentes, **caractérisé en ce que** le composé de formule IV est l'acide 2,5-furannedicarboxylique, l'acide 2,5-tetrahydrofurannedicarboxylique, l'acide 2-furoïque, l'acide 2,5-furannedicarboxylique monométhylester ou les acides 2,5-tetrahydrofurannedicarboxylique monométhylester

10. Procédé selon l'une des revendications précédentes, **caractérisé en ce qu'**il comprend une étape de récupération d'au moins le nitrile d'une part, et d'au moins l'imide cyclique 3-méthyl-glutarimide d'autre part, par séparation des composés du milieu réactionnel

11. Procédé selon la revendication 10, **caractérisé en ce qu'**au moins le nitrile récupéré est hydrogéné pour former l'amine correspondante.

12. Procédé selon la revendication 10 ou 11, **caractérisé en ce que** l'on fait réagir au moins l'imide cyclique 3-méthyl-glutarimide récupérée avec un alcool pour former le diester correspondant

## Patentansprüche

1. Verfahren zur Herstellung mindestens eines Nitrils der allgemeinen Formel I
(NC)ᵥ-R₁-(CN)_{w}
bzw. mindestens eines Nitrils der allgemeinen Formel III 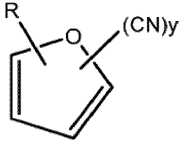 bzw. mindestens eines Nitrils der allgemeinen Formel V 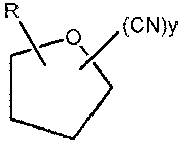 und mindestens des cyclischen Imids 3-Methylglutarimid
durch Reaktion zwischen mindestens einer Carbonsäure der allgemeinen Formel II
(HOOC)ₓ-R₁-(COOH)_{z}
bzw. mindestens einer Carbonsäure der allgemeinen Formel IV 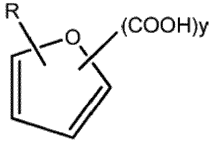 bzw. mindestens einer Carbonsäure der allgemeinen Formel VI 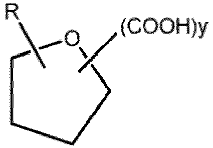 und mindestens 2-Methylglutaronitril (MGN) in Gegenwart eines Lewis-Säure-Katalysators,
wobei
x und z gleich 0 oder 1 sind, wobei (x+z) gleich 1 oder 2 ist,
v und w gleich 0 oder 1 sind, wobei (v+w) gleich 1 oder 2 ist,
R₁ für eine gesättigte oder ungesättigte, lineare oder verzweigte Kohlenwasserstoffgruppe, die Heteroatome umfassen kann, mit:
- 4 bis 34 Kohlenstoffatomen, wenn (x+z) gleich 2 ist,
- 2 bis 22 Kohlenstoffatomen, wenn (x+z) gleich 1 ist,
steht,
y gleich 1 oder 2 ist,
R einen oder mehrere Substituenten symbolisiert.

2. Verfahren nach Anspruch 1, **dadurch gekennzeichnet, dass** man ein Gemisch N von Dinitrilen, das 2-Methylglutaronitril (MGN), 2-Ethylsuccinonitril (ESN) und Adiponitril (AdN) umfasst, einsetzt.

3. Verfahren nach Anspruch 1, **dadurch gekennzeichnet, dass** es sich bei dem Gemisch N von Dinitrilen um ein Gemisch aus dem Verfahren zur Herstellung von Adiponitril durch doppelte Hydrocyanierung von Butadien handelt.

4. Verfahren nach Anspruch 2 oder 3, **dadurch gekennzeichnet, dass** das Gemisch N von Dinitrilen die folgende gewichtsbezogene Zusammensetzung aufweist:
Methyl-2-glutaronitril: zwischen 70% und 95%, vorzugsweise zwischen 80 und 85%,
Ethyl-2-succinonitril: zwischen 5% und 30%, vorzugsweise zwischen 8 und 12%,
Adiponitril: zwischen 0% und 10%, vorzugsweise zwischen 1 und 5%,
wobei der Rest auf 100% verschiedenen Verunreinigungen entspricht.

5. Verfahren nach einem der vorhergehenden Ansprüche, **dadurch gekennzeichnet, dass** die Verbindung der Formel II, IV und/oder VI aus einem erneuerbaren Stoff pflanzlichen oder tierischen Ursprungs stammt.

6. Verfahren nach einem der vorhergehenden Ansprüche, **dadurch gekennzeichnet, dass** die Verbindung der Formel II aus Capronsäure, Caprylsäure, Pelargonsäure, Caprinsäure, Laurinsäure, Myristinsäure, Palmitinsäure, Stearinsäure, Arachidinsäure, Adipinsäure, Heptandisäure, Octandisäure, Azelainsäure, Sebacinsäure, oder Undecandisäure, Dodecandisäure, Brassylsäure, Tetradecandisäure, Hexadecandisäure, Octadecandisäure, Eicosandisäure, Docosandisäure, Octadecensäure, Ölsäure, Ricinolsäure, Erucasäure, Linolsäure, Linolensäure und Fettsäuredimeren mit 36 Kohlenstoffatomen und Terephthalsäure ausgewählt ist.

7. Verfahren nach einem der vorhergehenden Ansprüche, **dadurch gekennzeichnet, dass** R aus:
- linearen oder verzweigten Alkylgruppen mit vorzugsweise 1 bis 6 Kohlenstoffatomen und noch weiter bevorzugt 1 bis 4 Kohlenstoffatomen,
- linearen oder verzweigten mono-, poly- oder perhalogenierten Alkylgruppen mit vorzugsweise 1 bis 6 Kohlenstoffatomen und 1 bis 13 Halogenatomen und noch weiter bevorzugt 1 bis 4 Kohlenstoffatomen und 1 bis 9 Halogenatomen,
- Ethergruppen R₂-O- oder Thioethergruppen R₂-S-, in denen R₂ für eine lineare oder verzweigte Alkylgruppe mit 1 bis 6 Kohlenstoffatomen und noch weiter bevorzugt 1 bis 4 Kohlenstoffatomen oder die Phenylgruppe steht,
- Acyloxy- oder Aroyloxygruppen R₂-CO-O-, in denen die Gruppe R₂ die oben angegebene Bedeutung besitzt,
Acyl- oder Aroylgruppen R₂-CO-O-, in denen die Gruppe R₂ die oben angegebene Bedeutung besitzt,
- der Hydroxylgruppe
- einem Halogenatom, vorzugsweise einem Fluoratom, ausgewählt ist.

8. Verfahren nach einem der Ansprüche 1 bis 6, **dadurch gekennzeichnet, dass** in der allgemeinen Formel IV oder VI y gleich 1 ist und R für -CO-O-R₃ steht, wobei R₃ für eine Alkylgruppe mit 1 bis 4 Kohlenstoffatomen steht.

9. Verfahren nach einem der vorhergehenden Ansprüche, **dadurch gekennzeichnet, dass** es sich bei der Verbindung der Formel IV um 2,5-Furandicarbonsäure, 2,5-Tetrahydrofurandicarbonsäure, Furan-2-carbonsäure, 2,5-Furandicarbonsäuremonomethylester oder 2,5-Tetrahydrofurandicarbonsäuremonomethylester handelt.

10. Verfahren nach einem der vorhergehenden Ansprüche, **dadurch gekennzeichnet, dass** es einen Schritt der Gewinnung mindestens des Nitrils einerseits und mindestens des cyclischen Imids 3-Methylglutarimid andererseits durch Abtrennung der Verbindungen aus dem Reaktionsmedium umfasst.

11. Verfahren nach Anspruch 10, **dadurch gekennzeichnet, dass** mindestens das gewonnene Nitril zu dem entsprechenden Amin hydriert wird.

12. Verfahren nach Anspruch 10 oder 11, **dadurch gekennzeichnet, dass** man mindestens das gewonnene cyclische Imid 3-Methylglutarimid mit einem Alkohol zu dem entsprechenden Diester umsetzt.

## Claims

1. Process for the preparation of at least one nitrile of general formula I
(NC)ᵥ-R₁-(CN)_{w}
or respectively of at least one nitrile of general formula III 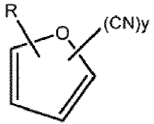 or respectively of at least one nitrile of general formula V 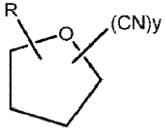 and of at least the cyclic imide 3-methylglutarimide, by reaction between at least one carboxylic acid of general formula II
(HOOC)ₓ-R₁-(COOH)_{z}
or respectively at least one carboxylic acid of general formula IV 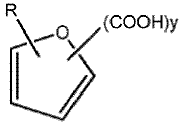 or respectively at least one carboxylic acid of general formula VI 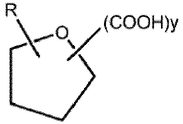 and at least 2-methylglutaronitrile (MGN), in the presence of a Lewis acid catalyst, with
x, z is equal to 0 or 1 with (x+z) equal to 1 or 2,
v, w is equal to 0 or 1 with (v+w) equal to 1 or 2,
R₁ represents a saturated or unsaturated and linear or branched hydrocarbon group which can comprise heteroatoms, comprising:
- from 4 to 34 carbon atoms when (x+z) is equal to 2,
- from 2 to 22 carbon atoms when (x+z) is equal to 1, y is equal to 1 or 2,
R symbolizes one or more substituents.

2. Process according to Claim 1, **characterized in that** a mixture N of dinitriles comprising 2-methylglutaronitrile (MGN), 2-ethylsuccinonitrile (ESN) and adiponitrile (AdN) is employed.

3. Process according to Claim 1, **characterized in that** the mixture N of dinitriles is a mixture resulting from the process for the manufacture of adiponitrile by double hydrocyanation of butadiene.

4. Process according to Claim 2 or 3, **characterized in that** the mixture N of dinitriles has the following composition by weight:
2-Methylglutaronitrile: between 70% and 95%, preferably between 80% and 85%,
2-Ethylsuccinonitrile: between 5% and 30%, preferably between 8% and 12%,
Adiponitrile: between 0% and 10%, preferably between 1% and 5%,
the remainder to 100% corresponding to various impurities.

5. Process according to one of the preceding claims, **characterized in that** the compound of formula II, IV and/or VI results from a renewable material of vegetable or animal origin.

6. Process according to one of the preceding claims, **characterized in that** the compound of formula II is chosen from caproic acid, caprylic acid, pelargonic acid, capric acid, lauric acid, myristic acid, palmitic acid, stearic acid, arachidic acid, adipic acid, heptanedioic acid, octanedioic acid, azelaic acid, sebacic or undecanedioic acid, dodecanedioic acid, brassylic acid, tetradecanedioic acid, hexadecanedioic acid, octadecanedioic acid, eicosanedioic acid, docosanedioic acid, octadecenoic acid, oleic acid, ricinoleic acid, erucic acid, linoleic acid, linolenic acid and fatty acid dimers comprising 36 carbon atoms, or terephthalic acid.

7. Process according to one of the preceding claims, **characterized in that** R is chosen from:
- linear or branched alkyl groups preferably having from 1 to 6 carbon atoms and more preferentially still from 1 to 4 carbon atoms,
- linear or branched mono-, poly- or perhalogenated alkyl groups preferably having from 1 to 6 carbon atoms and from 1 to 13 halogen atoms and more preferably still from 1 to 4 carbon atoms and from 1 to 9 halogen atoms,
- ether R₂-O- or thioether R₂-S- groups in which R₂ represents a linear or branched alkyl group having from 1 to 6 carbon atoms and more preferably still from 1 to 4 carbon atoms or the phenyl group,
- acyloxy or aroyloxy R₂-CO-O- groups in which the R₂ group has the meanings given above,
- acyl or aroyl R₂-CO- groups in which the R₂ group has the meanings given above,
- the hydroxyl group,
- a halogen atom, preferably a fluorine atom.

8. Process according to one of Claims 1 to 6, **characterized in that** y is equal to 1 and R represents -CO-O-R₃ with R₃ representing an alkyl group having from 1 to 4 carbon atoms in the general formula IV or VI.

9. Process according to one of the preceding claims, **characterized in that** the compound of formula IV is 2,5-furandicarboxylic acid, 2,5-tetrahydrofurandicarboxylic acid, 2-furoic acid, 2,5-furandicarboxylic acid monomethyl ester or 2,5-tetrahydrofurandicarboxylic acid monomethyl esters.

10. Process according to one of the preceding claims, **characterized in that** it comprises a stage of recovery, on the one hand, of at least the nitrile and, on the other hand, of at least the cyclic imide 3-methylglutarimide, by separation of the compounds of the reaction medium.

11. Process according to Claim 10, **characterized in that** at least the recovered nitrile is hydrogenated in order to form the corresponding amine.

12. Process according to Claim 10 or 11, **characterized in that** at least the recovered cyclic imide 3-methylglutarimide is reacted with an alcohol in order to form the corresponding diester.
